# EUROPÄISCHE PATENTSCHRIFT

(11) **EP 1 054 649 B1**
(45) Veröffentlichungstag und Bekanntmachung des Hinweises auf die Patenterteilung: **10.09.2003**
(21) Anmeldenummer: 99910238.7
(22) Anmeldetag: 19.02.1999
(51) Int. Cl.: A61F 2/06

(54) **VORRICHTUNG ZUM EINSETZEN EINES ROHRSTÜCKFÖRMIGEN IMPLANTATS IN EIN GEFÄSS**
DEVICE FOR INSERTING A TUBULAR IMPLANT INTO A VESSEL
PROCEDE POUR PLACER UN IMPLANT TUBULAIRE DANS UN VAISSEAU

(30) Priorität: 21.02.1998 DE 19807354
(43) Veröffentlichungstag der Anmeldung: 29.11.2000
(73) Patentinhaber: Aesculap AG & Co. KG, 78532 Tuttlingen (DE)
(72) Erfinder: DWORSCHAK, Manfred, D-78589 Dürbheim (DE); LUTZE, Theodor, D-78582 Balgheim (DE); STALLFORTH, Harald, D-78532 Tuttlingen (DE); WEIK, Thomas, D-78532 Tuttlingen (DE)
(74) Vertreter: Böhme, Ulrich, Dr. Dipl.-Phys.
(86) Internationale Anmeldenummer: EP9901073
(87) Internationale Veröffentlichungsnummer: WO99042058

(56) Entgegenhaltungen:
- WO-A-98/09583
- WO-A-98/33462
- US-A- 4 681 110
- US-A- 5 713 907

## Beschreibung

Die Erfindung betrifft eine Vorrichtung zum Einsetzen eines rohrstückförmigen Implantats in ein Gefäß mit einem mit einer Vorschubhandhabe versehenen, in das Implantat einschiebbaren Einführkopf, an dessen Umfang radial nach außen bewegbare Spreizelemente angeordnet sind, und mit einem am Einführkopf verschiebbar gelagerten Sperrorgan, das über ein Übertragungsorgan verschiebbar ist zwischen einer Ruhestellung, in der die Spreizelemente radial zurückgezogen sind, und einer Spreizstellung, in der die Spreizelemente radial nach außen vorstehen.

Die chirurgische Behandlung von abdominalen oder auch thorakalen Aortenaneurysmen erfolgt üblicherweise durch einen großen Bauch- oder Thoraxschnitt, wobei der aneurysmatische Gefäßanteil ausgeklemmt, reseziert und mit manueller Naht durch ein entsprechendes Prothesenstück ersetzt wird.

Es sind auch Operationsmethoden bekannt, bei denen endoluminal ein rohrstückförmiges Implantat in ein Gefäß eingeführt und dort festgelegt wird, beispielsweise mit Hilfe von Widerhaken (US-5 527 355 A). Derartige endoluminale Techniken sind weit weniger belastend als die konventionellen chirurgischen Eingriffe, allerdings ergeben sich dabei auch erhöhte Risiken. Es besteht nämlich die Gefahr, daß sich die eingesetzten rohrstückförmigen Implantate im Gefäß verschieben, außerdem können im Bereich der proximalen und der distalen Fixierung der rohrstückförmigen Implantate im Gefäß Undichtigkeiten auftreten.

Aus der US 5 713 907 A ist eine Vorrichtung der eingangs beschriebenen Art bekannt, bei welcher das Implantat durch Aufspreizen an einem Gefäß festlegbar ist. Es ist jedoch nicht möglich, mit dieser Vorrichtung das Implantat während des gesamten Einführweges sicher zu halten.

Ausgehend von diesem Stand der Technik liegt der Erfindung die Aufgabe zugrunde, eine Vorrichtung der gattungsgemäßen Art so auszugestalten, daß beim Einführen des Einführkopfes und des über diesen gezogenen Implantates dieses dauerhaft am Einführkopf gehalten werden kann, daß nach der Festlegung des Implantates am Gefäß jedoch der Einführkopf aus dem Operationsbereich zurückgezogen werden kann, ohne das Implantat mitzunehmen, und daß dies leicht und sicher realisiert werden kann.

Diese Aufgabe wird bei einer Vorrichtung der eingangs beschriebenen Art erfindungsgemäß dadurch gelöst, daß der Einführkopf eine lösbare Haltevorrichtung für das Implantat aufweist, die als eine flexible, um das Implantat herumgeführte und dieses gegen den Einführkopf spannende Bandage ausgebildet ist, deren Spannung herabsetzbar ist.

Mittels einer solchen Vorrichtung kann ein rohrstückförmiges Implantat auf den Einführkopf aufgeschoben und zusammen mit diesem in ein Gefäß eingeführt werden. Sobald die Einsetzposition erreicht ist, können durch eine Verschiebung des am Einführkopf gelagerten Sperrorgans die Spreizelemente in die Spreizstellung verfahren werden. Dadurch drücken sie nicht nur das den Einführkopf umgebende Implantat radial nach außen gegen die Innenwand des Gefäßes, sondern sie weiten auch Gefäß und Implantat im Anlagebereich aus, so daß zwischen dem Einführkopf einerseits und dem Implantat und der Gefäßwand andererseits ein Zwischenraum entsteht. Dieser Zwischenraum kann vom Operateur genutzt werden, um von außen her eine Fixierung des Implantats an der Gefäßwand vorzunehmen, beispielsweise durch Setzen einer umlaufenden Naht oder durch das Einführen von geeigneten Verbindungselementen. Dabei ist auch wesentlich, daß die beiden flächig aneinander liegenden Lagen des Implantats und der Gefäßwand durch die ausgefahrenen Spreizelemente gespannt werden, diese Spreizelemente wirken somit als Widerlager, so daß die beiden miteinander zu verbindenden Lagen vom Chirurgen in diesem vom Einführkopf abgehobenen Bereich mit einer Verbindung versehen werden können, ohne daß die Gefahr besteht, daß die Lagen sich bei der Bearbeitung verschieben oder ausweichen.

Durch die als Haltevorrichtung wirkende spannende Bandage wird sichergestellt, daß beim Einführen des Einführkopfes und des über diesen gezogenen Implantates dieses dauerhaft am Einführkopf gehalten ist, nach der Festlegung des Implantates am Gefäß jedoch kann durch Lösen der Haltevorrichtung der Einführkopf aus dem Operationsbereich zurückgezogen werden, ohne das Implantat mitzunehmen.

Insbesondere kann vorgesehen sein, daß die Bandage als Faden ausgebildet ist.

Dabei ist vorteilhaft, wenn die Enden der Bandage längs des Einführinstrumentes und längs der Handhabe frei verschiebbar geführt sind, insbesondere, wenn sie durch den Einführkopf hindurchgeführt sind. Es ist für den Operateur dann möglich, an der Austrittsstelle der Vorrichtung aus dem Gefäß die freien Enden der Bandage zu spannen, um die Haltevorrichtung zu schließen, oder die freien Enden locker zu lassen, um die Haltevorrichtung zu lösen.

Selbstverständlich können die freien Enden auch mit einem Betätigungsorgan verbunden sein, das beispielsweise längs der Vorrichtung verschiebbar gelagert ist.

Günstig ist es dabei, wenn der Einführkopf an seiner Außenfläche im Bereich der Bandage eine Vertiefung aufweist. Dadurch wird die Bandage längs des Einführkopfes festgelegt, außerdem kann dadurch erreicht werden, daß die Bandage radial nach außen nicht oder nur geringfügig über den Umfang des Einführkopfes vorsteht.

Insbesondere kann diese Vertiefung als Umfangsnut ausgebildet sein.

Der Einführkopf wird vorzugsweise als kreiszylindrischer Körper ausgebildet. Es ist günstig, wenn der Einführkopf an seinem der Vorschubhandhabe gegenüberliegenden vorderen Ende atraumatisch abgerundet ist, beispielsweise kann der Einführkopf eine annähernd kugelkalottenartige Form aufweisen.

Die Vorschubhandhabe kann nach Art eines Katheters ausgebildet sein, insbesondere kann sie durch ein flexibles Schubkräfte übertragendes Rohr ausgebildet sein.

Die Spreizelemente können unterschiedlich ausgebildet sein, es kann sich beispielsweise um flexible Füllkörper handeln, die durch Auffüllen mit einem Gas oder eine Flüssigkeit nach außen aufgeweitet werden, oder um mechanisch ausfahrbare Spreizelemente, die beispielsweise über einen Getriebemechanismus von der Ruhestellung in die Spreizstellung überführt werden können und umgekehrt.

Gemäß einer bevorzugten Ausführungsform ist vorgesehen, daß die Spreizelemente Federelemente sind, die in der entspannten Stellung radial nach außen gespreizt sind.

Dabei ist es günstig, wenn die Federelemente aus einer superelastischen Legierung bestehen, also aus einer Legierung aus der Gruppe der sogenannten Memory-Legierungen, die eine extreme elastische Verformbarkeit zeigen, beispielsweise kann es sich hierbei um eine NiTi-Legierung handeln.

Es ist dabei vorteilhaft, wenn die Federelemente in radial nach außen hin offenen Vertiefungen des Einführkopfes angeordnet sind und im entspannten Zustand aus diesen Vertiefungen hervorstehen. In der Ruhestellung sind diese Federelemente somit in den Einführkopf eingefahren und behindern nicht die Einführung des Einführkopfes und des auf diesem gehaltenen Implantates in das Gefäß.

Dabei kann weiterhin vorgesehen sein, daß das Sperrorgan in der Ruhestellung die Vertiefungen an ihrer Außenseite verschließt und die Federelemente dadurch in die Vertiefungen hineinschiebt.

Besonders vorteilhaft ist es dabei, wenn das Sperrorgan ein gegenüber dem Einführkopf längsverschiebliches Rohr ist, das in der einen Stellung die Vertiefungen verschließt, in der anderen Stellung die Vertiefungen dagegen freigibt.

Dabei kann vorgesehen sein, daß das Sperrorgan in der Ruhestellung die Außenfläche des Einführkopfes stetig fortsetzt, so daß das Rohr gleichzeitig eine Stützfläche für das auf den Einführkopf aufgezogene rohrstückförmige Implantat bildet.

Bei einer bevorzugten Ausführungsform sind die Federelemente parallel zur Längsrichtung des Einführkopfes einseitig an diesem festgelegte, im entspannten Zustand mit ihrem freien Ende radial nach außen abstehende Federzungen.

Diese können an ihren freien Ende atraumatisch verdickt und abgerundet sein, um Verletzungen des Implantates und/oder der Gefäßwand zu verhindern.

Günstig ist es, wenn mehrere derartige Federelemente längs des Umfanges des Einführkopfes verteilt sind, so daß das rohrstückförmige Implantat und die umgebende Gefäßwand in allen Richtungen gleichmäßig aufgeweitet werden. Der Chirurg kann dadurch die Festlegung des Implantates am Gefäß längs des gesamten Umfanges in gleicher Weise vornehmen.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht durch ein Gefäß mit eingeführtem Einführkopf, auf dem ein rohrstückförmiges Implantat gehalten ist, und
- Figur 2:: eine Längsschnittansicht eines Gefäßabschnittes mit einer Ausweitung und mit eingeschobenem Einführkopf und radial nach außen verformten Spreizelementen.

Das in der Zeichnung dargestellte chirurgische Instrument 1 zum Einführen eines rohrstückförmigen Implantates 2 umfaßt einen im wesentlichen kreiszylindrischen Einführkopf 3, der an einem katheterartigen, flexiblen Rohr 4 gehalten ist, das so lang ausgebildet ist, daß der Einführkopf 3 von einer Körperstelle aus, in der das Instrument in ein Körpergefäß eingeführt wird, bis zur Applikationsstelle im Innern des Körpers vorgeschoben werden kann.

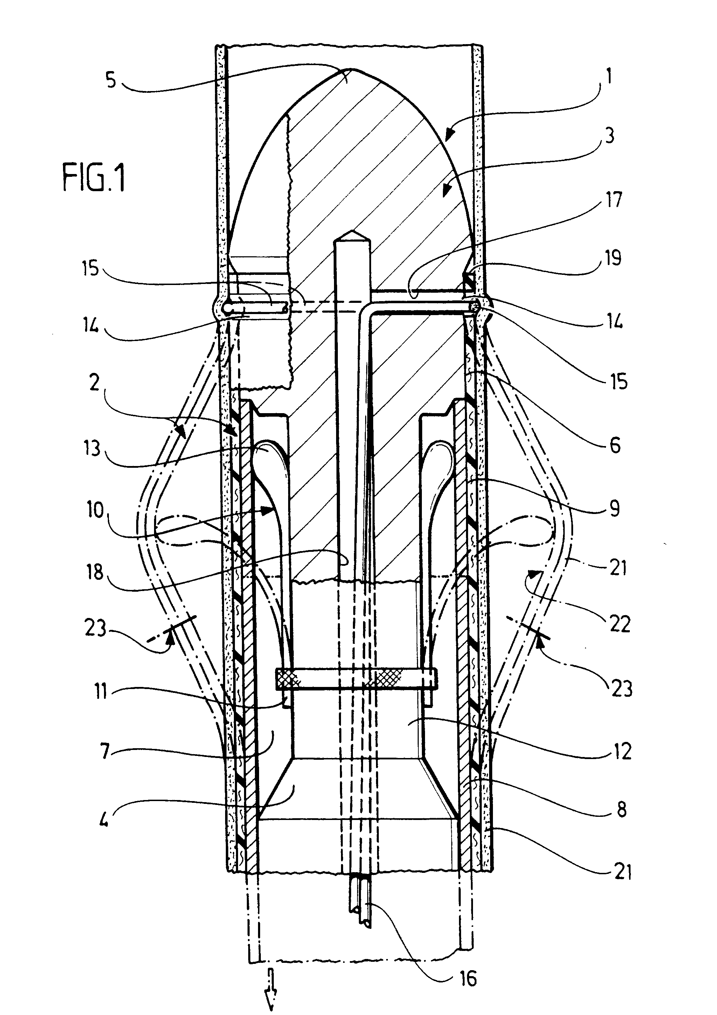

Der Einführkopf 3 ist im wesentlichen kreiszylindrisch ausgebildet und an seinem dem Rohr 4 gegenüberliegenden Ende 5 abgerundet, beispielsweise etwa kugelkalottenförmig. Dieses abgerundete Ende 5 steht dabei in radialer Richtung geringfügig über die zylindrische Außenfläche 6 des Einführkopfes 3 vor (Figur 1).

In dem kreiszylindrischen Bereich des Einführkopfes 3 weist dieser eine gegenüber dem Außenumfang zurückgesetzte, umfangsnutähnliche Vertiefung 7 auf, die durch ein auf dem Einführkopf 3 längsverschieblich gelagertes Rohr 8 verschließbar ist. Im verschlossenen Zustand schließt die Außenfläche 9 des Rohres 8 stetig an die Außenfläche 6 des Einführkopfes 3 an, so daß eine gemeinsame kreiszylindrische Umfangsfläche des Einführkopfes 3 ausgebildet wird.

Das Rohr 8 kann aus dieser die Vertiefung 7 verschliessenden Ruhestellung in eine offene Stellung verschoben werden, in der die Vertiefung 7 radial nach außen freigegeben wird, diese Stellung des Rohres 8 wird aus nachfolgend erörterten Gründen als Spreizstellung bezeichnet.

Die Verschiebung des Rohres 8 kann durch geeignete Übertragungsmittel erfolgen, beispielsweise kann sich das Rohr 8 bis an das Ende des Rohres 4 erstrecken, es ist dabei auch möglich, daß das Rohr 8 übergeht in ein Schub- und Zugelement, das längs des Rohres 4 bis zu dessen Ende hin verläuft, dies ist in der Zeichnung nicht näher dargestellt.

In der Vertiefung 7 sind am Boden 12 derselben parallel zur Längsrichtung des Einführkopfes 3 verlaufend eine Anzahl von Federzungen 10 an einem Ende 11 so festgelegt, daß die Federzungen 10 längs des Umfanges der Vertiefung 7 gleichmäßig verteilt sind. Die Federzungen 10 bestehen aus einem elastischen Material, insbesondere aus einer Superlegierung, und sind so geformt, daß die Federzungen 10 im entspannten Zustand mit ihren freien Enden 13 aus der Vertiefung 7 herausstehen und radial nach außen über die Kontur des Einführkopfes vorstehen (in Figur 1 gestrichelt dargestellt). Die freien Enden 13 der Federzungen sind verdickt und abgerundet ausgebildet.

Wenn sich das Rohr 8 in der zurückgezogenen Spreizstellung befindet, können sich alle Federzungen 10 in der beschriebenen Weise entspannen und radial nach außen über den Einführkopf 3 hervorstehen, verschiebt man das Rohr 8 in die vorgeschobene Schließstellung, zwingt das Rohr 4 alle Federzungen 10 entgegen ihrer Federkraft in das Innere der Vertiefung 7 hinein, so daß bei vollständig vorgeschobenem Rohr 8 die Federzungen 10 vollständig in der Vertiefung 7 aufgenommen werden (Figur 1, ausgezogene Linien).

Unmittelbar anschließend an das abgerundete Ende 5 des Einfuhrkopfes 3 weist dieser in der Außenfläche 6 eine Ringnut 14 auf, in die ein Faden 15 eingelegt ist. Die freien Enden 16 dieses Fadens 15 treten durch eine radiale Bohrung 17 in einen axialen Kanal 18 des Einführkopfes 3 ein, der in den Innenraum des Rohres 4 einmündet. Durch dieses Rohr 4 verlaufen die freien Enden 16 bis zum Ende des Rohres 4, so daß der Chirurg am Ende des Rohres 4 durch Ziehen an den beiden freien Enden 16 den Faden 15 kräftig in die Ringnut 14 hineinspannen kann, während diese Spannung beim Loslassen der freien Enden 16 wieder gelöst wird.

Das beschriebene Instrument wird zum Einsetzen des Implantates 2 in folgender Weise eingesetzt:

Zunächst wird auf den Einführkopf 3 das rohrstückförmige Implantat 2 so aufgeschoben, daß es flächig an der Außenfläche 6 des Einführkopfes 3 und an der Außenfläche 9 des in der Schließstellung stehenden Rohres 8 anliegt und dabei mit seinem freien Rand 19 die Ringnut 14 überdeckt. Im Bereich dieser Ringnut 14 wird der Faden 15 um das Implantat 2 herumgelegt, durch einen Einschnitt im Implantat 2 in die radiale Bohrung 17 eingeführt und durch Zug an den freien Enden 13 so gespannt, daß das Implantat 2 am Einführkopf 3 festgelegt wird.

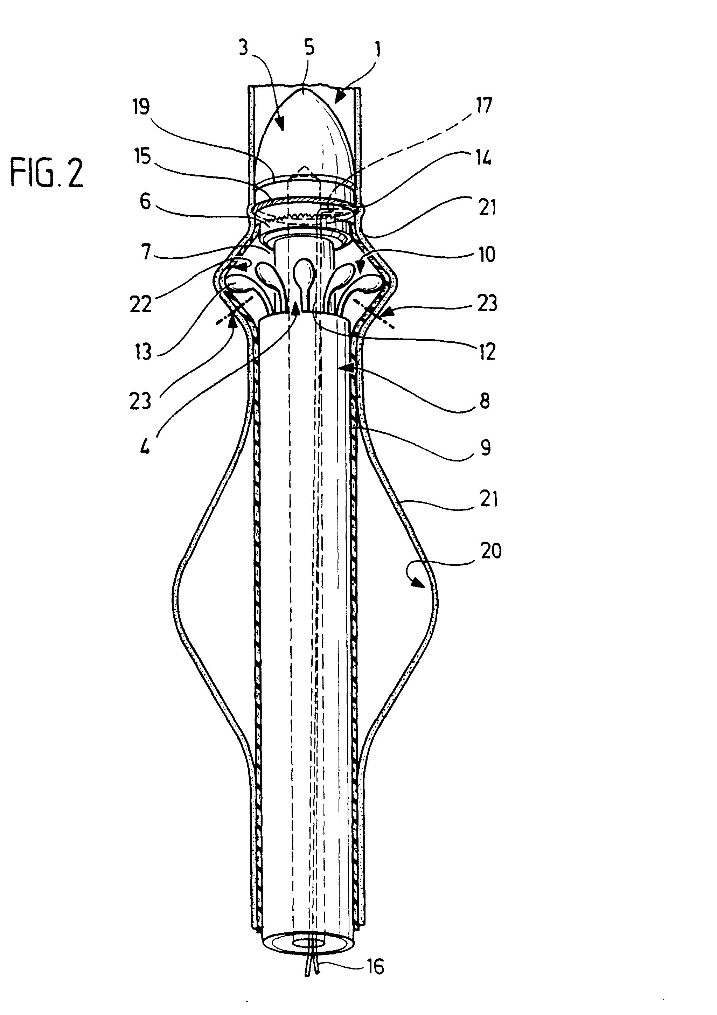

Der in dieser Weise vorbereitete Einführkopf 3 wird durch eine Öffnung des Gefäßes in das Gefäß eingeschoben und in diesem vorgeschoben bis zu der Stelle, an der das Implantat 2 eingesetzt werden soll. Dabei wird das Implantat 2 so positioniert, daß das Aneurysma 20 (Figur 2) überbrückt wird.

Sobald diese Position erreicht wird, zieht der Operateur das Rohr 8 in die Spreizstellung zurück, und dies führt dazu, daß die Federzungen 10 längs des gesamten Umfanges des Einführkopfes 3 sich radial nach außen entspannen und dabei sowohl das Implantat 2 als auch die Gefäßwand 21 elastisch aufweiten (strichpunktierte Linien in Figur 1). Sie heben dabei Implantat 2 und Gefäßwand 21 von der Außenfläche 6 des Einführkopfes 3 ab und bilden zwischen Einführkopf 3 und Implantat/Gefäßwand einen Ringraum 22 aus. Im Bereich des Ringraumes 22 spannen sie durch die elastische Aufweitung sowohl das Implantat 2 als auch die Gefäßwand 21 und ermöglichen so dem Chirurgen durch ein von außen an das Gefäß herangeführtes, in der Zeichnung nicht dargestelltes Instrument, Implantat 2 und Gefäßwand 21 dauerhaft und dicht miteinander zu verbinden, beispielsweise durch eine Umfangsnaht oder durch das Einführen von Haken oder anderen Befestigungsmitteln, die die beiden Lagen des Implantates 2 einerseits und der Gefäßwand 21 andererseits flächig miteinander verbinden. Die Verbindungsstelle ist in der Zeichnung mit dem Bezugszeichen 23 gekennzeichnet.

Es ist für die Verbindung von Bedeutung, daß das Implantat 2 im Bereich seines freien Randes 19 durch den Faden 15 festgelegt wird, im dahinterliegenden Bereich wird das Implantat dadurch fixiert, daß es über einen größeren Längenbereich an dem Rohr 8 außenseitig anliegt, dessen Außendurchmesser so groß ist, daß das Implantat 2 elastisch aufgeweitet wird. Diese beidseitige Fixierung des Implantates führt dazu, daß im Aufweitungsbereich, also im Bereich des Ringraumes 22, sowohl das Implantat als auch die Gefäßwand durch die Federzungen gespannt werden und nicht diese Spannung durch Nachrutschen wieder abgebaut wird. Diese Spannung ist neben der Schaffung des Ringraumes 22 für die einwandfreie Verbindung der beiden Lagen wichtig.

Sobald die Verbindung des Implantates 2 mit der Gefäßwand 21 an dem dem freien Rand 19 benachbarten Ende fertiggestellt ist, kann die Festlegung des Implantates 2 am Einführkopf 3 gelöst werden. Dies erfolgt durch Entspannen der freien Enden 16 des Fadens 15, der dann das Implantat 2 nicht mehr in die Ringnut 14 hineinspannt. Durch kurzes Vorschieben des Einführkopfes in der ursprünglichen Einschubrichtung gelangt der Faden 15 aus dem Anlagebereich der Außenseite des Implantates 2, so daß dann der Einführkopf 3 wieder zurückgezogen werden kann, der Faden 15 bleibt dann an der Innenseite des Implantates 2, das seinerseits durch die Verbindung mit der Gefäßwand 21 in seiner Position fixiert ist und dem Rückschieben des Einführkopfes 3 daher nicht folgt.

Der Einführkopf 3 wird jetzt nur so weit zurückgezogen, daß er auf der anderen Seite des Aneurysma 20 positioniert ist. In dieser Stellung wird das Rohr 8 erneut in die Spreizstellung verschoben, so daß die Federzungen 10 wieder nach außen gespreizt werden und erneut einen entsprechenden Ringraum ausbilden, in dem nun am gegenüberliegenden Ende des Implantates 2 in gleicher Weise eine Verbindung mit der Gefäßwand 21 hergestellt werden kann.

Nach Abschluß dieser Verbindung werden die Federzungen 10 durch Vorschieben des Rohres 8 erneut eingefahren, und dann kann der Einführkopf 3 wieder aus dem Gefäß entfernt werden.

Als Ergebnis dieser Operation erhält man ein in das Gefäß eingesetztes, das Aneurysma 20 überbrückendes Implantat 2, das an seinen beiden Enden längs seines Umfanges mit der umgebenden Gefäßwand 21 dauerhaft und dicht verbunden ist, so daß keine Gefahr besteht, daß das Implantat im Gefäß wandert oder daß der Bereich des Aneurysma 20 durch Undichtigkeiten weiter belastet wird.

## Patentansprüche

1. Vorrichtung zum Einsetzen eines rohrstückförmigen Implantats in ein Gefäß mit einem mit einer Vorschubhandhabe (4) versehenen, in das Implantat (2) einschiebbaren Einführkopf (3), an dessen Umfang (6) radial nach außen bewegbare Spreizelemente (10) angeordnet sind, und mit einem am Einführkopf (3) verschiebbar gelagerten Sperrorgan (8), das über ein Übertragungsorgan verschiebbar ist zwischen einer Ruhestellung, in der die Spreizelemente (10) radial zurückgezogen sind, und einer Spreizstellung, in der die Spreizelemente (10) radial nach außen vorstehen, **dadurch gekennzeichnet, daß** der Einführkopf (3) eine lösbare Haltevorrichtung für das Implantat (2) aufweist, die als eine flexible, um das Implantat (2) herumgeführte und dieses gegen den Einführkopf (3) spannende Bandage (15) ausgebildet ist, deren Spannung herabsetzbar ist.

2. Vorrichtung nach Anspruch 1, **dadurch gekennzeichnet, daß** die Bandage (15) als Faden ausgebildet ist.

3. Vorrichtung nach Anspruch 1, **dadurch gekennzeichnet, daß** die Enden (16) der Bandage (15) längs des Einführkopfes (3) und längs der Handhabe (4) frei verschiebbar geführt sind.

4. Vorrichtung nach Anspruch 3, **dadurch gekennzeichnet, daß** die Enden (16) der Bandage (15) durch den Einführkopf (3) hindurchgeführt sind.

5. Vorrichtung nach einem der Ansprüche 1 bis 4, **dadurch gekennzeichnet, daß** der Einführkopf (3) an seiner Außenfläche (6) im Bereich der Bandage (15) eine Vertiefung (14) aufweist.

6. Vorrichtung nach einem der voranstehenden Ansprüche, **dadurch gekennzeichnet, daß** der Einführkopf (3) ein kreiszylindrischer Körper ist.

7. Vorrichtung nach einem der voranstehenden Ansprüche, **dadurch gekennzeichnet, daß** der Einführkopf (3) an seinem der Vorschubhandhabe (4) gegenüberliegenden vorderen Ende (5) atraumatisch abgerundet ist.

8. Vorrichtung nach einem der voranstehenden Ansprüche, **dadurch gekennzeichnet, daß** die Vorschubhandhabe (4) durch ein flexibles, Schubkräfte übertragendes Rohr gebildet wird.

9. Vorrichtung nach einem der voranstehenden Ansprüche, **dadurch gekennzeichnet, daß** die Spreizelemente Federelemente (10) sind, die in der entspannten Stellung radial nach außen gespreizt sind.

10. Vorrichtung nach Anspruch 9, **dadurch gekennzeichnet, daß** die Federelemente (10) aus einer superelastischen Legierung bestehen.

11. Vorrichtung nach einem der Ansprüche 9 oder 10, **dadurch gekennzeichnet, daß** die Federelemente (10) in einer radial nach außen hin offenen Vertiefung (7) des Einführkopfes (3) angeordnet sind und im entspannten Zustand aus dieser Vertiefung (7) hervorstehen.

12. Vorrichtung nach Anspruch 11, **dadurch gekennzeichnet, daß** das Sperrorgan (8) in der Ruhestellung die Vertiefung (7) an ihrer Außenseite verschließt und die Federelemente (10) dadurch in die Vertiefung (7) hineinschiebt.

13. Vorrichtung nach Anspruch 12, **dadurch gekennzeichnet, daß** das Sperrorgan (8) ein gegenüber dem Einführkopf (3) längsverschiebliches Rohr ist.

14. Vorrichtung nach Anspruch 13, **dadurch gekennzeichnet, daß** das Sperrorgan (8) in der Ruhestellung die Außenfläche (6) des Einführkopfes (3) stetig fortsetzt.

15. Vorrichtung nach einem der Ansprüche 9 bis 14, **dadurch gekennzeichnet, daß** die Federelemente (10) parallel zur Längsrichtung des Einführkopfes (3) einseitig an diesem festgelegte, im entspannten Zustand an ihrem freien Ende (13) radial nach außen abstehende Federzungen sind.

16. Vorrichtung nach Anspruch 15, **dadurch gekennzeichnet, daß** die Federelemente (10) an ihrem freien Ende (13) atraumatisch verdickt und abgerundet sind.

17. Vorrichtung nach einem der Ansprüche 15 oder 16, **dadurch gekennzeichnet, daß** mehrere derartige Federelemente (10) längs des Umfanges des Einführkopfes (3) verteilt sind.

## Claims

1. A device for the placement of a tube-portion-shaped implant in a vessel, comprising an insertion head (3) provided with a feed handle (4) and adapted to be pushed into the implant (2), expanding elements (10), movable radially outwards, being arranged on the periphery (6) of the said insertion head, and comprising a barrier element (8), displaceably mounted on the insertion head (3), which is displaceable via a transfer element between an inoperative position in which the expanding elements (10) are retracted radially, and an expanded position in which the expanding elements (10) project radially outwards, **characterised in that** the insertion head (3) has a releasable retaining device for the implant (2) which is a flexible band (15) guided around the implant (2) and tightening the said implant against the insertion head (3), the tension of the said band being reducible.

2. A device according to Claim 1, **characterised in that** the band (15) is a thread.

3. A device according to Claim 1, **characterised in that** the ends (16) of the band (15) are guided so as to be freely displaceable along the insertion head (3) and along the handle (4).

4. A device according to Claim 3, **characterised in that** the ends (16) of the band (15) are guided through the insertion head (3).

5. A device according to one of Claims 1 to 4, **characterised in that** the insertion head (3) has a recess (14) on its exterior surface (6) in the region of the band (15).

6. A device according to one of the preceding Claims, **characterised in that** the insertion head (3) is a circular cylinder-shape body.

7. A device according to one of the preceding Claims, **characterised in that** the insertion head (3) is atraumatically rounded at its front end (5) opposite the feed handle (4).

8. A device according to one of the preceding Claims, **characterised in that** the feed handle (4) is formed by a flexible tube transferring pushing forces.

9. A device according to one of the preceding Claims, **characterised in that** the expanding elements are spring elements (10) which are expanded radially outwards in the released position.

10. A device according to Claim 9, **characterised in that** the spring elements (10) are made of a super-elastic alloy.

11. A device according to one of Claims 9 or 10, **characterised in that** the spring elements (10) are arranged in a recess (7) of the insertion head (3) which is open radially outwards, and protrude from this recess (7) in the released state.

12. A device according to Claim 11, **characterised in that** the barrier element (8) in the inoperative position closes the recess (7) on its exterior side and thereby pushes the spring elements (10) into the recess (7).

13. A device according to Claim 12, **characterised in that** the barrier element (8) is a tube which is displaceable longitudinally in relation to the insertion head (3).

14. A device according to Claim 13, **characterised in that** the barrier element (8) in the inoperative position continuously extends the exterior surface (6) of the insertion head (3).

15. A device according to one of Claims 9 to 14, **characterised in that** the spring elements (10) are flexible tongues fixed, parallel to the longitudinal direction of the insertion head (3), on one side to the said insertion head and projecting radially outwards at their free end (13) in the released state.

16. A device according to Claim 15, **characterised in that** the spring elements (10) are atraumatically thickened and rounded at their free end (13).

17. A device according to one of Claims 15 or 16, **characterised in that** several such spring elements (10) are distributed along the periphery of the insertion head (3).

## Revendications

1. Dispositif pour placer un implant de forme tubulaire dans un vaisseau, comprenant une tête d'introduction (3) dotée d'une manette (4) et susceptible d'être introduite dans l'implant (2), à la périphérie de laquelle (6) sont agencés des éléments d'écartement (10) déplaçables radialement vers l'extérieur, et comprenant un organe de blocage (8) monté avec faculté de déplacement sur la tête d'introduction (3), lequel est déplaçable via un organe de transmission entre une position de repos dans laquelle les éléments d'écartement (10) sont rétractés radialement, et une position écartée dans laquelle les éléments d'écartement (10) dépassent radialement vers l'extérieur, **caractérisé en ce que** la tête d'introduction (3) comporte un dispositif de maintien détachable pour l'implant (2), ce dispositif étant réalisé sous forme d'un bandage (15) flexible, passé autour de l'implant (2), et serrant celui-ci contre la tête d'introduction (3), dont le serrage peut être annulé.

2. Dispositif selon la revendication 1, **caractérisé en ce que** le bandage (15) est réalisé sous forme de fils.

3. Dispositif selon la revendication 1, **caractérisé en ce que** les extrémités (16) du bandage (15) sont menées avec faculté de déplacement libre le long de la tête d'introduction (3) et le long de la manette (4).

4. Dispositif selon la revendication 3, **caractérisé en ce que** les extrémités (16) du bandage (15) sont passées à travers la tête d'introduction (3).

5. Dispositif selon l'une des revendications 1 à 4, **caractérisé en ce que** la tête d'introduction (3) présente à sa surface extérieure (6) un renfoncement (14) dans la région du bandage (15).

6. Dispositif selon l'une des revendications précédentes, **caractérisé en ce que** la tête d'introduction (3) est un corps cylindrique droit.

7. Dispositif selon l'une des revendications précédentes, **caractérisé en ce que** la tête d'introduction (3) est arrondie de façon atraumatique à son extrémité antérieure (5) située à l'opposé de la manette d'avance (4).

8. Dispositif selon l'une des revendications précédentes, **caractérisé en ce que** la manette d'avance (4) est formée par un tube flexible qui transmet des forces de poussée.

9. Dispositif selon l'une des revendications précédentes, **caractérisé en ce que** les éléments d'écartement sont des éléments à ressort (10) qui sont écartés radialement vers l'extérieur dans la position détendue.

10. Dispositif selon la revendication 9, **caractérisé en ce que** les éléments à ressort (10) sont formés en alliage super élastique.

11. Dispositif selon l'une ou l'autre des revendications 9 et 10, **caractérisé en ce que** les éléments à ressort (10) sont agencés dans un renfoncement (7), ouvert radialement vers l'extérieur, de la tête d'introduction (3) est dépassent hors de ce renfoncement (7) dans l'état détendu.

12. Dispositif selon la revendication 11, **caractérisé en ce que** l'organe de blocage (8) referme le renfoncement (7) au niveau de sa face extérieure dans la position de repos, et repousse par conséquent les éléments à ressort (10) dans le renfoncement (7).

13. Dispositif selon la revendication 12, **caractérisé en ce que** l'organe de blocage (8) est un tube déplaçable longitudinalement par rapport à la tête d'introduction (3).

14. Dispositif selon la revendication 13, **caractérisé en ce que** l'organe de blocage (8) prolonge en continu la surface extérieure (6) de la tête d'introduction (3) dans la position de repos.

15. Dispositif selon l'une des revendications 9 a 14, **caractérisé en ce que** les éléments à ressort (10) sont des languettes à ressort fixé d'un côté sur la tête d'introduction (3) et parallèlement à la direction longitudinale de celle-ci, lesdites languettes dépassant à l'état détendu radialement vers l'extérieur à leur extrémité libre (13).

16. Dispositif selon la revendication 15, **caractérisé en ce que** les éléments à ressort (10) sont épaissis et arrondis de manière atraumatique à leur extrémité libre (13).

17. Dispositif selon l'une ou l'autre des revendications 15 et 16, **caractérisé en ce que** plusieurs éléments à ressort (10) de ce type sont répartis le long de la périphérie de la tête d'introduction (3).
